Europäisches Patentamt

European Patent Office

Office européen des brevets

(11) Veröffentlichungsnummer: **0 433 833 A1**

# (12) EUROPÄISCHE PATENTANMELDUNG

(21) Anmeldenummer: 90123718.0

(22) Anmeldetag: 10.12.90

(51) Int. Cl.5: **C12Q 1/04,** C12Q 1/10, G01N 31/22

(30) Priorität: 22.12.89 CH 4630/89

(43) Veröffentlichungstag der Anmeldung:
26.06.91 Patentblatt 91/26

(84) Benannte Vertragsstaaten:
**BE CH DE ES FR GB IT LI NL**

(71) Anmelder: **F. HOFFMANN-LA ROCHE AG**
**Postfach 3255**
**CH-4002 Basel(CH)**

(72) Erfinder: **Füllhaas, Jürgen, Dr.**
**Florastrasse 8**
**CH-4127 Birsfelden(CH)**
Erfinder: **Le Dain, Michel, Dr.**
**10 rue Jean Schoerlin, Neuwiller**
**F-68220 Hegenheim(FR)**

(74) Vertreter: **Kloter, Rudolf et al**
**Postfach 3255 Grenzacherstrasse 124**
**CH-4002 Basel(CH)**

(54) **Indolreagenz.**

(57) Die Erfindung betrifft ein trockenes Indolreagenz für Küvetten von Analysenautomaten. Es enthält p-Dimethylaminobenzaldehyd und eine bis zu mindestens 37°C feste Säure, nämlich ein- oder mehrfach sulfoniertes und gegebenenfalls niedrig alkyliertes Benzol oder Naphthalin.

Geeignete Säuren sind Toluol-4-sulfonsäure, Benzolsulfonsäure und Naphthalin-1,5-disulfonsäure. Besonders bevorzugt ist Naphthalin-trisulfonsäure.

EP 0 433 833 A1

## INDOLREAGENZ

Für die Identifizierung von Enterobakterien (und Nonfermentern) ist der Nachweis der Bildung von Indol ein wichtiger Test. Die übliche Durchführung des Testes besteht darin, dass nach der Inkubationszeit das Indolreagenz zu der zu prüfenden Kultur zugegeben wird. Dies gilt für die Röhrchenteste und auch für viele automatisierte Teste, wo vor oder nach der photometrischen Auswertung manuell das Reagenz zugegeben werden, visuell beurteilt und via Tastatur das Ergebnis eingegeben werden muss.

Aufgabe der vorliegenden Erfindung ist es, ein Indolreagenz zu schaffen, das voll in einen Analysenautomaten integriert werden kann, so dass keine manuellen Schritte mehr notwendig sind.

Bisher bekannte Indolreagenzien liegen nur in flüssiger Form vor. Als Säure wird in der Regel Salzsäure verwendet. Als Reagenz wird p-Dimethylaminobenzaldehyd verwendet. Für die Verwendung in der Küvette eines Analysenautomaten ist eine flüssige Form des Reagenz nicht geeignet.

Es stellte sich demnach die Aufgabe, nach einer Formulierung zu suchen, die flüssig in die Küvette abgefüllt und dann getrocknet werden kann. Aus diesem Grunde kommen nur bei Raumtemperatur feste Säuren in Betracht.

Die Bedingung, bei Raumtemperatur feste Säure, muss von zusätzlichen Kriterien vervollständigt werden um ein brauchbares Indolreagenz zu erhalten. Die Säure muss stark sein (ein pH-Wert von ungefähr 0,1 - 0,8), zudem muss sie gut wasserlöslich sein und mindestens 1 g p-Dimethylaminobenzaldehyd pro 100 ml Säure auflösen. Weiterhin muss sie nach Zugabe der Kultur sofort löslich sein. Im weiteren muss die Indolreaktion so rasch als möglich erfolgen (innerhalb weniger Sekunden).

Im Rahmen der vorliegenden Erfindung wurde nun überraschend ein derartiges trockenes Indolreagenz gefunden. Die Erfindung betrifft demnach ein trockenes Indolreagenz für Küvetten von Analysenautomaten enthaltend p-Dimethylaminobenzaldehyd und eine bis zu mindestens 37° C feste Säure nämlich ein- oder mehrfach sulfoniertes und gegebenenfalls niedrig alkyliertes Benzol oder Naphthalin.

Das Indolreagenz enthält den p-Dimethylaminobenzaldehyd in einer Konzentration von 1-5%, vorzugsweise 2,5%.

· Als geeignete Säure haben sich Toluol-4-sulfonsäure, Benzol-sulfonsäure, Benzol-1,2-disulfonsäure, Naphthalin-1,5-disulfonsäure und Naphthalin-trisulfonsäure erwiesen. wobei die letztere besonders bevorzugt ist.

Die vorerwähnten Säure sind alle bekannt und kommerziellerhältlich. Bei der Naphthalin-trisulfonsäure handelt es sich um eine solche, die bis zu 90% an 1,3,6-Naphthalintrisulfonsäure enthält. Da diese Säuren üblicherweise in Form der Salze erhältlich sind, werden die Säuren nach an sich bekannten Methoden z.B. durch Kationenaustausch freigesetzt.

Die obenerwähnten Säuren werden in der Regel in einer Molarität zwischen 0,1 und 1, besonders bevorzugt von 0,5 eingesetzt. Das Indolreagenz gemäss der vorliegenden Erfindung wird vorzugsweise in der Küvette eines Analysenautomaten vorfabriziert.

Diese Vorfabrikation erfolgt dadurch, dass man den p-Dimethylaminobenzaldehyd in einer Lösung der Säure löst, in eine Küvette einbringt und anschliessend unterhalb des Schmelzpunktes der Säure trocknet.

Bevorzugt wird die Säure in Wasser gelöst, wobei diese Lösung in die Küvette eingebracht und getrocknet wird.

In der Praxis wird der Indoltest so durchgeführt, dass in die Küvette mit dem vorfabrizierten Indolreagenz eine gewisse Menge Keimsuspension und Nährlösung zugegeben wird, worauf nach dem Auflösen des Indolreagenzes und nach der Indolreaktion 2 Absorptionsmessungen durchgeführt werden. Aus der Absorptionsdifferenz kann auf das Vorliegen Indol produzierender Bakterien geschlossen werden.

Beispiel

In einer für die photometrische Messung oder visuelle Beurteilung geeignete Küvette werden 20 Mikroliter einer wässrigen Lösung von Naphthalin-1,3,6 Trisulfonsäure (0,5 M) und 4-Dimethylaminobenzaldehyd (2,5%) dosiert und in einem Tunnel Trocknungsofen bei 60° C getrocknet. Im vorliegenden Beispiel enthält die Küvette nach dem Trocknungsprozess 4,183 Milligramm Indol-Trockenreagenz.

Für den Nachweis von Indol-bildenden Enterobakterien (und Nonfermentern) werden 85 Mikroliter einer Keimsuspension aus gewissen optischen Dichten (z.B. Mac Farlane 1) und 20 Mikroliter eines Nährmediums inkubiert und anschliessend in die das Indol-Trockenreagenz enthaltende Küvette transferiert, in welcher nach Auflösung des Reagenzes die Nachweisreaktion in Abhängigkeit des durch die Bakterien gebildeten Indols stattfindet.

Zur Auswertung werden die bei den Wellenlängen 546 nm und 616 nm gemessenen Absorptionen voneinander subtrahiert. Die Grenze liegt bei 75 Milliabsorbanz Einheiten (Schichtdicke 4mm). Ueber dieser Grenze sind die Proben Indolpositiv.

**Ansprüche**

punktes der Säure trocknet.

1. Trockenes Indolreagenz für Küvetten von Analysenautomaten enthaltend p-Dimethylaminobenzaldehyd und eine bis zu mindestens 37° C feste Säure, nämlich ein- oder mehrfach sulfoniertes und gegebenenfalls niedrig alkyliertes Benzol oder Naphthalin.

2. Indolreagenz nach Patentanspruch 1, dadurch gekennzeichnet, dass der p-Dimethylaminobenzaldehyd in einer Konzentration von 1-5% vorliegt.

3. Indolreagenz nach Anspruch 1 oder 2, dadurch gekennzeichnet, dass der p-Dimethylaminobenzaldehyd in einer Konzentration von 2,5% vorliegt.

4. Indolreagenz nach einem der Ansprüche 1 - 3, dadurch gekennzeichnet, dass die Säure Toluol-4-sulfonsäure ist.

5. Indolreagenz nach einem der Ansprüche 1 - 3, dadurch gekennzeichnet, dass die Säure Benzol-sulfonsäure ist.

6. Indolreagenz nach einem der Ansprüche 1 - 3, dadurch gekennzeichnet, dass die Säure Benzol-1,2-disulfonsäure ist.

7. Indolreagenz nach einem der Ansprüche 1 - 3, dadurch gekennzeichnet, dass die Säure Naphthalin-1,5-disulfonsäure ist.

8. Indolreagenz nach einem der Ansprüche 1 - 3, dadurch gekennzeichnet, dass die Säure Naphthalin-trisulfonsäure ist.

9. Indolreagenz nach einem der Ansprüche 4 - 8, dadurch gekennzeichnet, dass die Säuren eine Molarität von 0,1-1 aufweisen.

10. Indolreagenz gemäss einem der Ansprüche 4 - 9, dadurch gekennzeichnet, dass die Säuren eine Molarität von 0,5 aufweisen.

11. Indolreagenz nach einem der Ansprüche 1 - 10, dadurch gekennzeichnet, dass es in einer Küvette eines Analysenautomaten vorfabriziert vorliegt.

12. Verfahren zur Herstellung eines Indolreagenzes gemäss Anspruch 11, dadurch gekennzeichnet, dass man den p-Dimethylaminobenzaldehyd in einer Lösung der Säure gemäss den Ansprüche 4 - 8 löst, in eine Küvette einbringt und anschliessend unterhalb des Schmelz-

**Europäisches Patentamt**

# EUROPÄISCHER RECHERCHENBERICHT

Nummer der Anmeldung

## EP 90 12 3718

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | GB-A-1 182 605 (MILES LABORATORIES INC.)<br>* Seite 1, Zeilen 68-81; Seite 2, Zeilen 15-46 *<br>– – – | 1-12 | C 12 Q<br>1/04<br>C 12 Q 1/10<br>G 01 N 31/22 |
| A | US-A-3 634 198 (A. TRUMAN)<br>* Das ganze Dokument *<br>– – – | 1-12 | |
| Y | G. PAIK: "Reagents, stains and miscellaneous test procedures", Sektion XI, Kapitel 98, E.H. LENNETTE (ed.): "Manual of clinical microbiology", Auflage 3, 1980, Seien 1000-1024, American Society for Microbiology, Washington, DC<br>* Seite 1003, die ganze rechte Spalte - Seite 1004, Zeile 7 *<br>– – – – | 1-12 | |

| RECHERCHIERTE SACHGEBIETE (Int. Cl.5) |
|---|
| C 12 Q<br>G 01 N |

Der vorliegende Recherchenbericht wurde für alle Patentansprüche erstellt

| Recherchenort | Abschlußdatum der Recherche | Prüfer |
|---|---|---|
| Den Haag | 11 März 91 | DOEPFER K-P. |